# EUROPEAN PATENT SPECIFICATION

(11) **EP 2 191 766 B1**
(45) Date of publication and mention of the grant of the patent: **07.09.2011**
(21) Application number: 09011588.2
(22) Date of filing: 10.09.2009
(51) Int. Cl.: A61B 1/00

(54) **Device for preventing slippage of protective covers and an endoscope set**
Vorrichtung zur Verhinderung des Gleitens von Schutzabdeckungen und Endoskopsatz
Dispositif pour empêcher le glissement de couvercles protecteurs et ensemble d'endoscope

(30) Priority: 28.11.2008 JP 2008303344
(43) Date of publication of application: 02.06.2010
(73) Proprietor: Tyco Healthcare Group LP, Mansfield, MA 02048 (US)
(72) Inventor: Yamada, Yoshitaka, Shizuoka 437-0004 (JP)
(74) Representative: Olsson, Carl H.S.

(56) References cited:
- EP-A- 1 759 625
- EP-A- 1 905 478
- WO-A-00/77428
- DE-U1- 20 212 767
- US-A- 3 822 052
- US-A- 4 369 794

## Description

### Field of Technology:

This invention concerns a device for preventing slippage of protective covers, the purpose of which is to fix protective covers, which cover endoscopes and prevent them from being soiled, in a state in which slippage with respect to the endoscopes is prevented, and an endoscope set which is provided with this device.

### Background Technology:

Up to now, long endoscopes have been used, for example, for diagnosing and treating patients. In order to spare the trouble of cleaning and sterilizing them after their use, protective covers are used to cover the endoscopes. Moreover, certain slippage preventing devices and fixing means are used to fix these protective covers in a state in which slippage with respect to the endoscopes is prevented (see, for example, JP 2000-041938). These disposable fiberscope covers (protective covers) consist of a transparent cover tip, a long, slender insertion part, a cover fixing part, which has a larger diameter than the insertion part, and a fixing part which is formed on the end of the cover fixing part.

When a fiberscope (endoscope) is covered with this disposable fiberscope cover, the cover tip and the insertion part of the disposable fiberscope cover are put on the fiberscope and the cover fixing part is then pulled onto the end of the disposable fiberscope cover and the fixing part is fixed to the outside of the fiberscope with the fiberscope fixing part (device for preventing slippage of protective covers). Tube clamps incorporating rigid pressure exerting members for closing flexible tubes are known, for example, in EP 1905 478 A2.

### Disclosure of Invention:

However, the fiberscope fixing parts for fixing the aforementioned disposable fiberscope covers of the prior art to the fiberscopes are formed corresponding to the fixing parts of the disposable fiberscope covers. Therefore, if disposable fiberscope covers with different shapes are used, the fiberscope fixing parts used must also be ones which correspond to these differently shaped disposable fiberscope covers. Furthermore, the fiberscope may be damaged if the fixing part of the disposable fiberscope cover presses strongly against the fiberscope.

In addition, the aforementioned disposable fiberscope cover is formed in a shape which conforms to the outer shape of the fiberscope, and the fixing part on its end is fixed in a particular fixing position on the outer surface of the fiberscope by the fiberscope fixing part. Therefore, if fiberscopes with different shapes are used, the disposable fiberscope covers that are used must be ones which correspond to the differently shaped fiberscopes.

Therefore, the problem arises that it is easy to make mistakes with the dimensions of the fiberscopes, the disposable fiberscope covers, and the fiberscope fixing parts of the covers. There is also the problem that, if the cover tips are not tightly fitted to the tips of the fiberscopes, the clarity of the images obtained by the fiberscopes is reduced, but the operation of fitting the cover tip tightly to the tip of the fiberscope is difficult to perform.

This invention was made to deal with these problems. Its purpose is to provide a device for preventing slippage of protective covers which can fix the protective covers which cover endoscopes in the correct state, without slippage, without damaging the endoscopes or the covers, and an endoscope set which is provided with this device.

In order to accomplish this purpose, one characteristic of the make-up of the device of this invention for preventing slippage of protective covers is that it is a device for preventing the slippage of protective covers, the purpose of which is to fix protective covers for endoscopes in a state in which slippage with respect to the endoscopes is prevented, and it is provided with a flexible, plate-shaped main body both ends of which can be joined by bending it; a pair of insertion holes which are made in the main body with a distance between them and through which an endoscope which is covered by the protective cover is inserted; and a flexible pressing part which is placed on the inner surface of the main body and which is arranged to deform and presses the endoscope which is covered by the protective cover onto another part in the inner surface of the main body when the ends of the main body are joined.

In the device of this invention for preventing slippage of protective covers, the main body is formed from a flexible plate-shaped member and is made in such a way that its ends can be joined by bending or curving it. Furthermore, it is provided with a pair of insertion holes with a distance between them and a flexible pressing part on its inner surface, such that, when the ends are joined, the endoscope, which is covered by a protective cover which is inserted through the insertion holes, can be pressed by the pressing part onto another part on the inner surface of the main body, that is, a part which opposes the pressing part on the inner surface of the main body. In this case, the protective cover is tightly fitted to the endoscope by the pressure of the pressing part. Therefore, the protective cover is fixed to the endoscope in a state in which it is prevented from slipping from the endoscope, when a force is exerted in such a way that the protective cover slips with respect to the endoscope, by the friction between the protective cover and the endoscope.

Since the protective cover is fixed, in this way, in a state in which it is prevented from slipping with respect to the endoscope by the pressing part, which presses the endoscope covered by the protective cover onto a part opposite to the pressing part on the inner surface of the main body, there are no particular limits on the shape of the protective cover which covers the endoscope. That is, the length of the protective cover need not match the length of the endoscope, and the rear end of the protective cover need not have a shape which corresponds to a specific part which is placed on the outer surface of the endoscope. The protective cover may merely be any cover which can cover the necessary parts of the endoscope. Furthermore, since the protective cover is fixed in a state in which it is prevented from slipping with respect to the endoscope by the pressure of the pressing part, which is flexible, the pressing part flexes when the endoscope, which is covered with the protective cover, is pressed by the strong force of the pressing part, and the endoscope and the protective cover are not damaged.

Furthermore, as the pair of insertion holes through which the endoscope, covered by the protective cover, is inserted, it is desirable for at least one insertion hole to be constituted by a hole with an inner diameter somewhat larger than the outer diameter of the endoscope covered by the protective cover, so that the endoscope covered by the protective cover does not wobble very much, and for the other insertion hole to be constituted by a hole into which the endoscope covered by the protective cover can be inserted with some leeway. By doing so, the insertion holes can be easily placed in opposite positions when the main body is flexed and the endoscope covered by the protective cover can be passed through the insertion holes in the main body in an approximately straight fashion.

Moreover, another characteristic of the make-up of the device of this invention for preventing slippage of protective covers is that the main body is formed from a base and a engaging piece and a engaged piece, which extend from the sides of the base; hook-shaped engaging pieces which can engage are placed on the front ends of the engaging piece and engaged piece; the pair of insertion holes are placed on the boundary part between the base and the engaging piece and the boundary part between the base and the engaged piece in the main body; the pressing part is placed on at least one of the engaging piece and the engaged piece, or the base; and when the engaging pieces have been engaged, the pressing part presses the endoscope which is covered by the protective cover onto the base or at least one of the engaging piece and the engaged piece.

In the device of this invention for preventing slippage of protective covers, the main body is formed from a base and a engaging piece and a engaged piece; it is made in such a way that the engaging parts on their ends are engaged by bending the engaging piece and the engaged piece towards the base. Furthermore, insertion holes are made in the boundary parts between the base and the engaging piece and the base and the engaged piece and a flexible pressing part is placed on at least one of the engaging piece and the engaged piece. When the engaging pieces have been engaged, the pressing part presses the endoscope which is covered by the protective cover onto the base or at least one of the engaging piece and the engaged piece.

In this way, the operation of fixing the protective cover is facilitated, since the protective cover can be fixed in a state in which slippage with respect to the endoscope is prevented by passing the endoscope covered with the protective cover through the pair of insertion holes and engaging the hook-shaped engaging parts placed on the ends of the engaging piece and the engaged piece. Furthermore, the pressing part in this case can be placed on either the engaging piece, the engaged piece, or the base, or on both the engaging and engaged pieces.

Moreover, still another characteristic of the make-up of the device of this invention for preventing slippage of protective covers is that a projecting part is formed in a part of the inner surface of the main body which opposes the pressing part, and the endoscope which is covered by the protective cover is pressed into contact with the projecting part by the pressure of the pressing part. By doing so, the protective cover can be fixed more reliably in a state in which it is prevented from slipping with respect to the endoscope, since the endoscope covered with the protective cover is pressed from both sides. Furthermore, it is desirable to form the surface on the projecting part which touches the protective cover into a smooth, curved surface. By doing so, the endoscope and the protective cover can be more reliably prevented from being damaged by touching the pressing part. Furthermore, the projecting part may be formed as a rigid or a flexible part.

Moreover, still another characteristic of the make-up of the device of this invention for preventing slippage of protective covers is that the pressing part is formed by a flexible piece the tip of which is formed as a free end. By doing so, the endoscope and the protective cover can be more reliably prevented from being damaged by touching the pressing part, since the pressing part is bent in one direction and its side surface, rather than its end surface, touches the protective cover when the endoscope covered with the protective cover is pressed by the pressing part. Furthermore, in this invention, the statement that the tip is a free end means that the tip is in a state in which it is not held by something else and can be swung by applying a force.

Moreover, still another characteristic of the make-up of the device of this invention for preventing slippage of protective covers is that the pressing part is formed by a flexible piece both ends of which are fixed to the inner surface of the main body and the center part of which is curved and projects outward. By doing so, the endoscope and the protective cover can be more reliably prevented from being damaged by touching the pressing part, since the pressing part is bent so that it follows the protective cover and this bent surface touches the protective cover when the endoscope covered with the protective cover is pressed by the pressing part.

Moreover, still another characteristic of the make-up of the device of this invention for preventing slippage of protective covers is that the pressing part is formed from a resin. In this case, the pressing part can be formed by forming it integrally with the main body, or it can be formed as a separate body and fixed onto the main body. For example, if it is formed from a flexible piece which extends from at least one of the engaging piece and the engaged piece or the base, the pressing part can be formed by molded resin body which is made by integral molding with the main body. Moreover, if the pressing part is formed from a flexible piece which has a curved central part and both ends of which are fixed to at least one of the engaging piece and the engaged piece or the base, it can be formed by molded resin body which is made by integral molding with the main body, or both ends of the pressing part which is formed from a separate resin body can be fixed to at least one of the engaging piece and the engaged piece or the base. By doing so, the pressing part can be easily molded.

Moreover, still another characteristic of the make-up of the endoscope set of this invention is that it comprises the endoscope, the protective cover, and any of the devices for preventing slippage of protective covers mentioned above. By doing so, any desired endoscope and protective cover can be selected, and they can be combined with the device for preventing slippage of protective covers and used as an endoscope set.

### Best Embodiments for Working the Invention:

### First Working Embodiment:

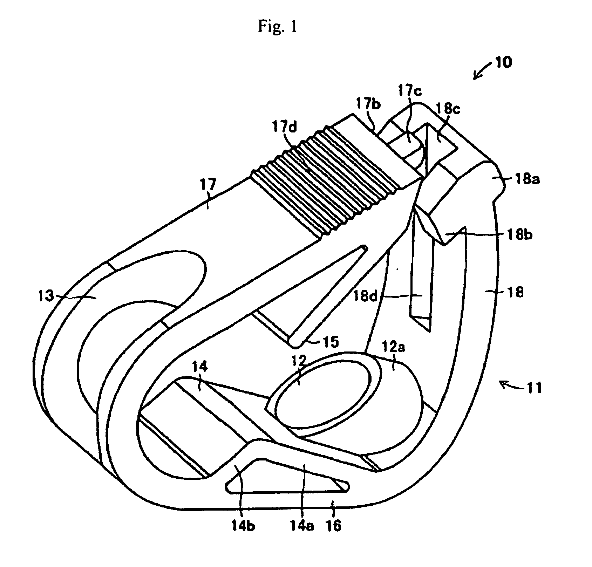

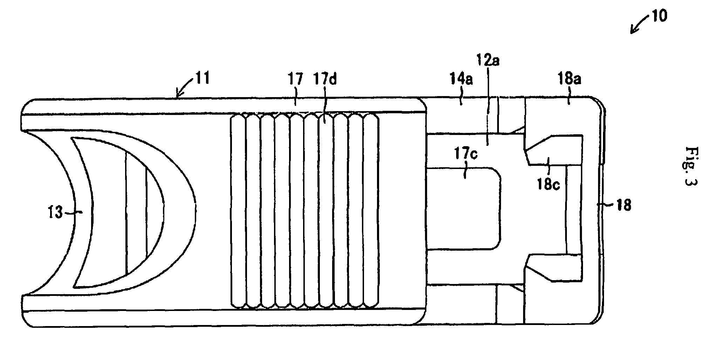

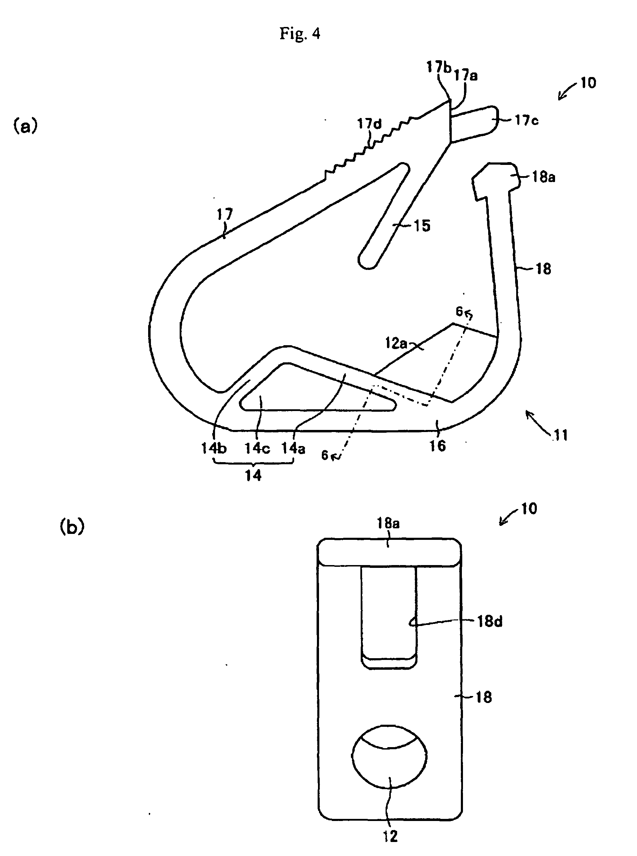

The first working embodiment of this invention will be explained below by using the drawings. Figs. 1 to 5 show a device for preventing slippage of protective covers 10 of this working embodiment. This device for preventing slippage of protective covers 10 is formed by integrally molding an ABS resin (an acrylonitrile-butadiene-styrene copolymer synthetic resin); it is made up of a main body 11, which is formed by bending a long, slender, flexible plate so that its side surface (Fig. 4 (a)) is approximately triangular, and placing insertion holes 12 and 13, a projecting part 14, and a pressing part 15 on it. The main body 11 is made up a base 16, located in the center of the long, slender plate (the bottom part in the state in which it is shown in Figs. 1 to 5), and an engaging piece 17 and an engaged piece 18, located on the sides of the base 16 in the longitudinal direction; the boundary part between the base 16 and the engaging piece 17 and the base 16 and the engaged piece 18 are both bent outward to form projecting arcs.

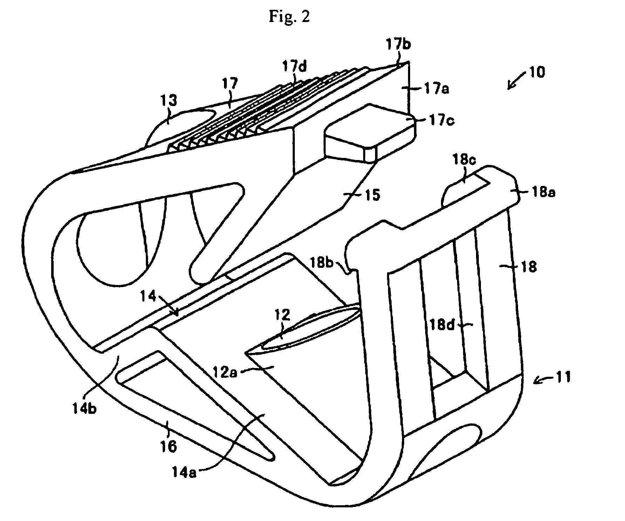

The engaging piece 17, in the state in which a force is not applied to it and it is not deformed, extends from one end of the base 16 so that it forms an angle of approximately 45 degrees with respect to the base 16. Furthermore, as shown in Fig. 2 and Fig. 4(a), in the state in which it is seen from the side, an inclined surface 17a, the upper part of which forms an obtuse angle with the upper surface of the engaging piece 17 and the lower part of which forms an acute angle with the lower surface of the engaging piece 17 is formed at the tip of the engaging piece 17. An engaging part 17b is formed by the acute angle between this inclined surface 17a and the engaging piece 17. Moreover, a guiding projection 17c which functions as a horizontal stop and a guide is placed on the central part, in the horizontal direction, of the lower side of the included surface 17a. A corrugated part 17d for preventing slipping, consisting of numerous projections and grooves perpendicular to the longitudinal direction (the transverse direction) of the engaging piece 17, is formed on the upper surface of the tip end of the engaging piece 17.

The engaged piece 18 is formed from a shorter piece than the engaging piece 17; in the state in which it is not deformed by applying a force, it extends from the other end of the base 16 so that it inclines toward the engaging piece 17 at somewhat more than a right angle with the base 16. Moreover, a bulging part 18a, which swells more in the thickness direction than the lower part, when seen from the side, is formed on the end (upper end) of the engaged piece 18. An engaged part 18b which can engage with the engaging part 17b of the engaging piece 17 is formed on the engaging piece 17 side, on the lower end part of this bulging part 18a. This engaged part 18b is formed by a concave part, into which the engaging part 17b fits from below.

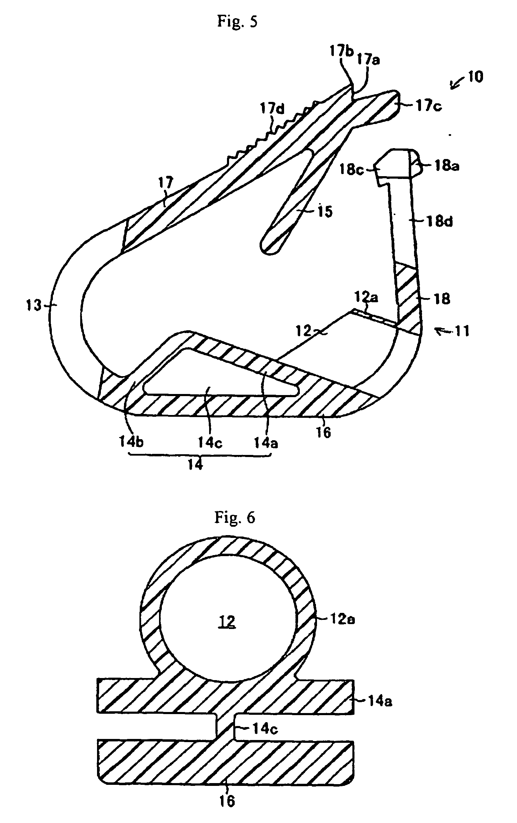

Furthermore, a concave part 18c, which extends from the upper end of the bulging part 18a continuously over approximately the center, in the longitudinal direction, of the engaged piece 18, and a hole 18d are formed in the part of the bulging part 18a on the side of the engaging piece 17, in the center, in the width direction, of the engaged piece 18, and the part of the engaged piece 18 extending downward from the bulging part 18a. Therefore, the engaged part 18b is formed so that it is divided into both sides in the width direction of the engaged piece 18 by the boundary part of the concave part 18c and the hole 18d. The concave part 18c is a concave part which is formed in the parts on both sides of the bulging part 18a and the right-side part (the part of the cross section shown in Fig. 5); its width on its upper side is greater than the width on its lower side. The guiding projection 17c can pass up and down through this concave part 18c. When the guiding projection 17c passes through the concave part 18c, the concave part 18c biases the guiding projection 17c to the center in the width direction.

The hole 18d is a window-shaped through hole which is formed in the part which extends from the lower end of the bulging part 18a in the engaged piece 18 to somewhat below the center of the engaged piece 18 in the longitudinal direction; the guiding projection 17c can move up and down in the state in which it has passed through this hole 18d. Therefore, when the corrugated part 17d of the engaging piece 17 is pressed downward, the guiding projection 17c presses on the bulging part 18a, and goes down into the concave part 18c so that it is guided onto the inner surface of the concave part 18c, while the engaged piece 18 is pressed and spread outward. Moreover, when the guiding projection 17c passes through the lower end part of the concave part 18c, it fits into the hole 18d and in this way the engaging part 17b and the engaged part 18b are engaged.

The insertion hole 12 is formed in the tubular insertion part 12a which is formed on the inner surface of the curved part of the boundary between the base 16 and the engaged piece 18. The tubular insertion part 12a is formed from a short tubular body which extends toward the inside of the main body 11 from the boundary between the base 16 and the engaged piece 18, and is situated in such a way that it is inclined with its tip placed higher than the base end on the boundary part of the base 16 and the engaged piece 18. Moreover, the tip end surface of the tubular insertion part 12a is formed in a surface inclined so that its opening faces somewhat upward. Furthermore, the insertion hole 12 is formed by a hole which passes from the outer surface of the boundary part between the base 16 and the engaged piece 18 through to the opening at the end of the tubular insertion part 12a.

The insertion hole 13 is formed from a long oval through hole which is formed in the part from the curved part of the boundary part between the base 16 and the engaging piece 17 to approximately the center of the engaging piece 17 in the longitudinal direction. This insertion hole 13 is disposed so that it is opposite the insertion hole 12; it is formed so that the insertion hole 13 is located on an extension line of the insertion hole 12, even when the engaging piece 17 and the engaged piece 18 are further bent toward the base 16 from their normal state.

The projecting part 14 is formed on the upper surface of the base 16; its shape, seen from the side, is approximately triangular. That is, the projecting part 14 is formed from an inclined part 14a, which extends in an upward inclined direction from the engaged piece 18 side of the base 16 toward the engaging piece 17 side, so that it holds the lower part of the tubular insertion part 12a; an inclined part 14b which is shorter than the inclined part 14a and extends from the end of the inclined part 14a to the engaging piece 17 side of the base 16; and a reinforcing wall part 14c, which is placed in the center in the width direction of the base 16 and the inclined parts 14a and 14b and the shape of which is triangular, as seen from the side. Moreover, the upper surface of the connecting part of the inclined parts 14a and 14b is a smooth curved curved surface. A cross section of the tubular insertion part 12a and the engaging piece 18 side part of the projecting part 14 in Fig. 4 (a) is shown in Fig. 6. Furthermore, Fig. 5 shows a cross section in front of the reinforcing wall part 14c in Fig. 4 (a).

The pressing part 15 is formed from a flexible projecting piece which extends toward the upper end side of the projecting part 14 from the lower surface of the tip of the engaging piece 17. This pressing part 15 extends in such a way that its angle with the engaging piece 17 is about 30 degrees; its tip is formed as a free end, and is shaped as a smooth curve. Therefore, when the engaging piece 17 and the engaged piece 18 are bent so that the engaging part 17b and the engaged part 18b are engaged, the pressing part 15 is inclined so that its lower surface (the surface on the engaged piece 18 side) is opposite the projecting part 14. Furthermore, the widths of the projecting part 14 and the pressing part 15 are the same as the width of the main body 11.

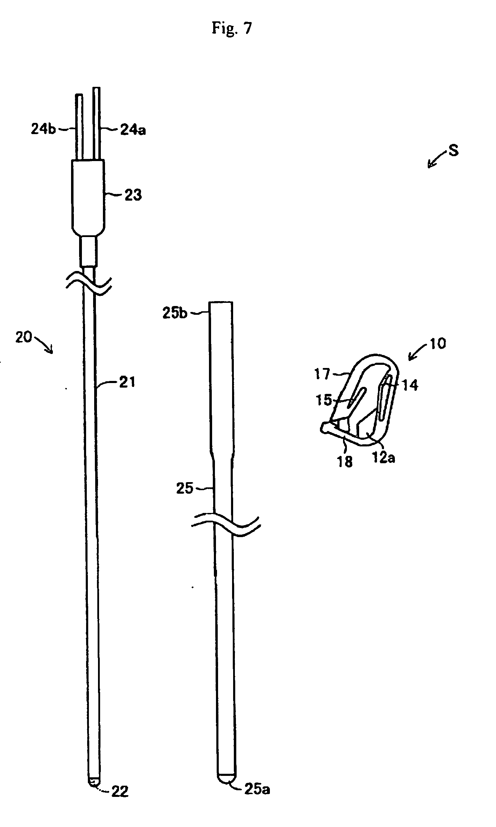

The device for preventing slippage of protective covers 10 formed in this way is used to fix a protective cover 25 on an endoscope 20, shown in Fig. 7, so that slippage is prevented. An endoscope set S is formed from the device for preventing slippage of protective covers 10, the endoscope 20, and the protective cover 25. The endoscope 20 is formed from a long fiberscope shaft 21, a lens 22 attached to the tip of the fiberscope shaft 21, and a connecting part 23 attached to the base end of the fiberscope shaft 21; its total length is set at 930 mm. A drawing of the inner part is omitted, but the fiberscope shaft 21 is constituted by multiple light guides for shining a light and a fiber bundle, consisting of an image guide for transmitting an image through the lens 2, covered by a surface covering layer which consists of a nylon resin and a urethane resin; it is flexible.

The connecting part 23 connects a wire 24a, which connects the image guide to an image display device (not shown) and a wire 24b which connects the light guide to a light source device (not shown). The lens 22 sends an image, obtained by shining the light guide, to the image display device through the image guide and the wire 24a. That is, the light guide shines the light which is sent from the light source device on the object, making it observable, and the image guide sends the reflected light from the object, focused by the lens, to the image display device. Furthermore, the image display device enlarges the image which is sent and displays it on the image display part with which it is provided.

Moreover, the tip of the protective cover 25 is closed by a transparent window 25a; it is formed by a flexible tube made of PP (polypropylene) resin, PE (polyethylene) resin, PUR (polyurethane) resin, PVC (polyvinyl chloride) resin, etc., the base end 25b on the open side of which has a somewhat larger diameter than the other part. This protective cover 25 is formed with a width which can cover the fiberscope shaft 21; its total length is set at 500 mm, and the length of its base end 25b is set at 150-160 mm. This protective cover 25 can cover the lens 22 and approximately half of the fiberscope shaft 21, on the tip end, in the endoscope 20.

In addition, the lens 22 of the fiberscope shaft 21 is caused to contact the inner surface of the window 25a tightly, and the endoscope 20 can be made to display a good image on the image display part in a state in which the tip part is prevented from being soiled by attaching the protective cover 25 to the endoscope 20. Furthermore, since the base end 25b of the protective cover 25 is wider than the other part, the operation of attaching the protective cover 25 to the endoscope is facilitated by holding this base end 25b.

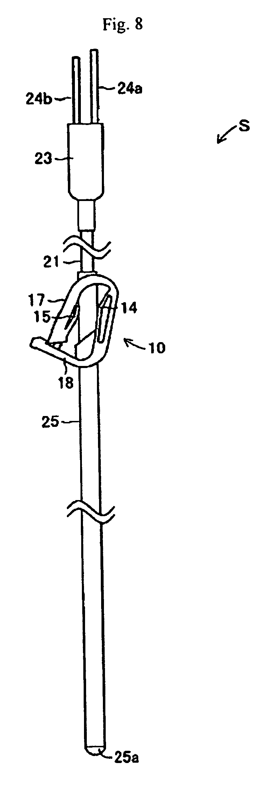

Furthermore, as shown in Fig. 8, by attaching the device for preventing slippage of protective covers 10 to the central part (the base end part of the protective cover 25) of the fiberscope shaft 21 on the endoscope 20, the tip end of which is covered with the protective cover 25, the protective cover 25 is fixed to the endoscope 20 in a state in which slippage is prevented. In this case, the tip of the endoscope 20, covered with the protective cover 25, is inserted into the main body 11 of the device for preventing slippage of protective covers 10 from the insertion hole 13 and its tip is projected outside the main body 11 from the insertion hole 12. Furthermore, after the device for preventing slippage of protective covers 10 is moved to the base end 25b of the protective cover 25, the concave part 17d of the engaging piece 17 is pressed downward and the engaging part 17b and the engaged part 18b are engaged.

In this way, the central part of the fiberscope shaft 21 of the endoscope 20, covered with the protective cover 25, is pressed to the projecting part 14 side by the pressing part 15 and the protective cover 25 is made to closely contact the fiberscope shaft 21 by this pressure. Therefore, when a force is exerted on the fiberscope shaft 21 in such a way as to make the protective cover 25 slip, the protective cover 25 is fixed to the fiberscope shaft 21 by the friction between the protective cover 25 and the fiberscope shaft 21, in a state in which it is prevented from slipping from the fiberscope shaft 21. At this time, the fiberscope shaft 21, covered by the protective cover 25, is kept in a straight form, without bending. Moreover, since the pressing part 15 is flexible, no excessive force is applied to the protective cover 25 and the fiberscope shaft 21. Therefore, damage to the protective cover 25 and the fiberscope shaft 21 can be prevented.

The endoscope set S formed in this manner is used, for example, to confirm the location of a gastric fistula catheter (not shown), which is placed in a hole made in the abdominal wall and stomach wall of a patient. In this case, the part of the endoscope 20 covered by the protective cover 25 is inserted into the gastric fistula catheter and its tip is projected from the lower end of the gastric fistula catheter and placed in the stomach. Moreover, its light passes through the wire 24b and the fiberscope shaft 21 and shines on the stomach wall.

Furthermore, the reflected light from the stomach wall, which is illuminated by the light from the light guide, is focused by the lens 22 and then sent to the image display device through the image guide and the wire 24a of the fiberscope shaft 21. Since the image set to the image display device is displayed enlarged on the image display part of the image display device, whether the tip of the gastric fistula catheter is placed in a correct state in the stomach can be confirmed from the image displayed on this image display part. Furthermore, although the explanation is omitted, the endoscope 20 has the function of being able to correct the direction of the lens 22 by rotating the tip end part. In this way, all of the surfaces inside the stomach and the tip of the gastric fistula catheter can be seen.

If it is confirmed that the gastric fistula catheter is place in the correct state, the operations of removing the endoscope set S, in the assembled state, from the gastric fistula catheter and removing the protective cover 25 from the endoscope 20 are performed. First, the endoscope set S is withdrawn from the gastric fistula catheter by pulling it from the patient's body and the device for preventing slippage of protective covers 10 is removed from the protective cover 25, etc. The removal of the device for preventing slippage of protective covers 10 in this case is facilitated since it can be performed simply by pressing the corrugated part 17d of the engaging piece 17 downward with the thumb of the hand holding the endoscope 20 while the bulging part 18a of the engaged piece 18 is pressed so that it is separated from the engaging piece 17 with the tip of this thumb.

Next, the protective cover 25 is removed by pulling it from the fiberscope shaft 21. The protective cover 25 is discarded and the endoscope 20 and the device for preventing slippage of protective covers 10 are used in the next opportunity. At this time, the lens 22 and the fiberscope shaft 21 are not soiled, since they have not touched body fluids or residues in the patients body and stomach, and they hardly need any treatment, such as cleaning or sterilization. Moreover, when the endoscope 20 is reused, the fiberscope shaft 21 is covered with a new protective cover 25, and this protective cover is fixed in a state in which it is prevented from slipping on the fiberscope shaft 21 by the device for preventing slippage of protective covers 10.

Thus, the device for preventing slippage of protective covers 10 consists of a main body 11, a base 16, an engaging piece 17 provided with an engaging part 17b, and an engaged piece 18 provided with an engaged part 18b which can engage with the engaging part 17b. Furthermore, it is so constituted that the fiberscope shaft 21, covered with the protective cover 25, can be inserted in an almost straight form into the insertion hole 13 which is placed in the boundary part between the base 16 and the engaging piece 17 in the main body 11 and the insertion hole 12 which is placed in the boundary part between the base 16 and the engaged piece 18. Moreover, a flexible pressing part 15 is placed on the lower surface of the engaging piece 17 and a projecting part 14 is placed on the upper surface of the base 16; when the engaging part 17b and the engaged part 18b are engaged, the fiberscope shaft 21, covered with the protective cover 25, is pressed onto the projecting part 14 by the pressing part 15 and the protective cover 25 can be fixed in a state in which it is prevented from slipping on the fiberscope shaft 21.

Thus, since the protective cover 25 is fixed in a state in which it is prevented from slipping on the fiberscope shaft 21, the protective cover 25 which covers the fiberscope shaft 21 need not correspond in length with the fiberscope shaft 21. That is, the length of the protective cover 25 may be that of the part of the fiberscope shaft 21 which needs to be covered the part which enters the body); it is not necessary to replace a protective cover 25 corresponding to the length of the fiberscope shaft 21, as long as this length alone is secured. Moreover, since the protective cover 25 is fixed in a state in which it is prevented from slipping on the fiberscope shaft 21 by the pressure of the flexible pressing part 15, the fiberscope shaft 21 and the protective cover 25 are prevented from being damaged.

Furthermore, since a guiding projection 17c is placed on the engaging piece 17 and a concave part 18c and a hole 18d are placed on the engaged piece 18, when the engaging part 17b and the engaged part 18b are engaged, the guiding projection 17c is guided to the concave part 18c and enters the hole 18d. Therefore, the engaging piece 17 does not slide sideways with respect to the engaged piece 18 and the engaging piece 17 and the engaged piece 18 are engaged in the correct state. Furthermore, since the fiberscope shaft 21 covered by the protective cover 25 is pressed and this pressure is relieved by forming the main body 11, which is flexible, in an approximately triangular shape and mounting and dismounting the engaging part 17b and the engaged part 18b formed on its ends, these mounting and dismounting operations are made easy. In addition, the device for preventing slippage of protective covers 10 can be easily molded, since it is formed by molding an ABS resin in one body.

### Second Working Embodiment:

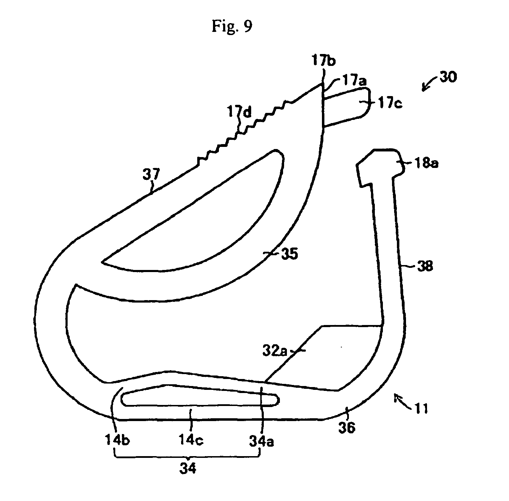

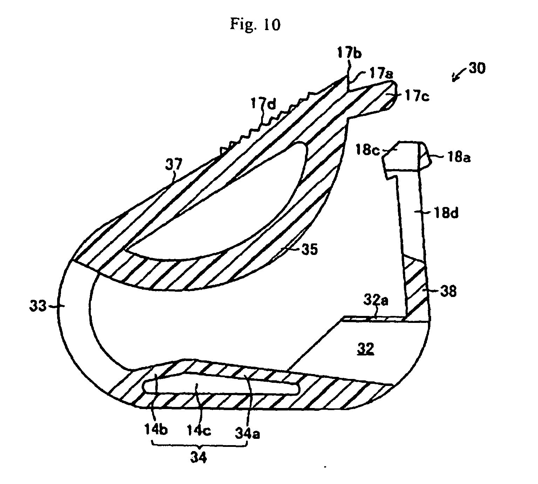

Figs. 9 and 10 show a device for preventing slippage of protective covers 30 of a second working embodiment of the invention. In this device for preventing slippage of protective covers 30, the pressing part 35 bridges between the tip part on the lower surface of the engaging piece 37 and the base 36 side of the lower surface of the engaging piece 37; it is made of a flexible piece which is curved so that it projects downward. This pressing part 35 projects its central part, in the longitudinal direction, downward when no force is applied, forming an arc which is rounded overall, but when the central part is pressed, this part is deformed so that it approaches a flat surface, and small-diameter arc-shaped parts are produced on both sides of the part which has become approximately flat.

Moreover, the insertion hole 33 is placed in the curved part of the boundary part between the base 36 and the engaging piece 37; its upper end extends as far as the part in which the lower end of the pressing part 35 is connected to the engaging piece 37. That is, this insertion hole 33 is formed by an oval through hole with a length shorter than the insertion hole 13 of the first working embodiment described above. Moreover, the projecting part 34 is formed in such a way that the height of the upper end of the insertion hole 33 is lower than the projecting part 14 of the first working embodiment mentioned above by the amount that it is lower than the height of the upper end part of the insertion hole 13.

The angle of inclination of the tubular insertion part 32a, inside of which the insertion hole 32 is formed, is made smaller than the angle of inclination of the tubular insertion part 12a of the first working embodiment described above, and the lower part of the tubular insertion part 32a is supported on the inclined part 34a of the projecting part 34. Therefore, the insertion holes 32 and 33 are placed opposite to each other and the insertion hole 33 is located on an extension line of the insertion hole 32 even when the engaging piece 37 and the engaged piece 38 are bent towards the base 36. Thus, the fiberscope shaft 21 covered by the protective cover 25 can be inserted into the insertion holes 32 and 33 while its approximately straight form is maintained. The make-up of the other parts of this device for preventing slippage of protective covers 30 is the same as in the device for preventing slippage of protective covers 10 of the first working embodiment described above. Therefore, the same symbols are placed on the same parts, and their explanation will be omitted.

This device for preventing slippage of protective covers 30, like the device for preventing slippage of protective covers 10 described above, is used to fix a protective cover 25 on the fiberscope shaft 21 of an endoscope 20 so that it is prevented from slipping. By means of this device for preventing slippage of protective covers 30, when the fiberscope shaft 21 covered with the protective cover 25 is pressed by the pressing part 35, the central part of the pressing part 35 is bent into an approximately flat shape, following the protective cover 25, and this bent, approximately flat part is pressed onto the protective cover 25, so that the fiberscope shaft 21 and the protective cover 25 can be more reliably prevented from being damaged by the pressure of the pressing part 35. The other efficacies of the action of this device for preventing slippage of protective covers 30 are the same as those of the device for preventing slippage of protective covers 10 of the first working embodiment described above.

Moreover, the device for preventing slippage of protective covers of this invention is not limited to the working embodiments described above; it can be suitably modified within the technical scope of this invention. For example, in the working embodiments described above, the pressing parts 15 and 35 are placed on the engaging pieces 17 and 37, but the pressing part can also be placed on the engaged piece or the base. If the pressing part is placed on the engaged piece, the engaged piece is made longer than the engaging piece, and the length of the hole in the vertical direction is shortened. Moreover, if the pressing part is placed on the base, the deformation of the pressing part is not regulated by the tubular insertion part. Furthermore, pressing parts can be placed on both the engaging piece and the engaged piece. In these cases, moreover, a projecting part can be placed on at least one of the engaging piece and the engaged piece.

Furthermore, one may also not include a projecting part, and form the device in such a way that the fiberscope shaft 21 covered by the protective cover 25 is pressed in the part where the pressing parts are opposite each other. Or, if a projecting part is included, the projecting part may be formed from a flexible part. Furthermore, in the working embodiments described above, the devices for preventing slippage of protective covers 10 and 30 are formed by integral molding, but the pressing part 35 in the second working embodiment can be formed as a separate body from the main body 11. The pressing part in this case may be formed from a molded resin body, or it may be formed from a thin metal plate with both ends fixed to the main body. Furthermore, as the resin material constituting the devices for preventing slippage of protective covers 10 and 30, besides an ABS resin, one can use a PP (polypropylene) resin, a PE (polyethylene) resin, a PC (polycarbonate) resin, etc.

Moreover, in the working embodiments described above, the main bodies 11 are formed from bases 16 and 36, engaging pieces 17 and 37, and engaged pieces 18 and 38, but the make-up of the main body is not limited to these cases; ones with various shapes can be used. For example, one can use a main body in which one end of an arc-shaped piece is connected to an end of a flat base, or one in which the ends of a pair of arc-shaped pieces are connected to each other.

### Simple Explanation of Drawings:

- Fig. 1: Perspective view showing a device for preventing slippage of protective covers of the first working embodiment of this invention
- Fig. 2: Perspective view showing the state of the device for preventing slippage of protective covers of Fig. 1 seen from anther direction
- Fig. 3: Top view of a device for preventing slippage of protective covers of the first working embodiment of this invention
- Fig. 4: Device for preventing slippage of protective covers of the first working embodiment of this invention; (a) side view, (b) front view
- Fig. 5: Cross sectional view of a device for preventing slippage of protective covers of the first working embodiment
- Fig. 6: Cross sectional view 6-6 of Fig. 4 (a)
- Fig. 7: Exploded view of an endoscope set
- Fig. 8: Front view showing the endoscope set in the assembled state
- Fig. 9: Side view showing a device for preventing slippage of protective covers of the second working embodiment of this invention
- Fig. 10: Cross sectional view of a device for preventing slippage of protective covers of the second working embodiment

### Explanation of Symbols:

10, 30 ... devices for preventing slippage of protective covers, 11 ... main body, 12, 13, 32, 33 ... insertion holes, 14, 34 ... projecting parts, 15, 35 ... pressing parts, 16, 36 ... bases, 17, 37 ... engaging pieces, 17b ... engaging part, 18, 38 ... engaged pieces, 18b ... engaged part, 20 ... endoscope, 25 ... protective cover, S ... endoscope set

## Claims

1. A device (10, 30) for preventing slippage of protective covers for endoscopes with respect to said endoscopes, the device being provided with:
a flexible, plate-shaped main body (11) both ends of which can be joined by bending it; and
a pair of insertion holes (12, 13) which are made in said main body with a distance between them and through which an endoscope which is covered by said protective cover can be inserted;
**characterized in that** the device further comprises:
a flexible pressing part (15, 35) which is placed on the inner surface of said main body and which is arranged to deform and press the endoscope which is covered by said protective cover onto another part (14) in the inner surface of said main body when the ends of said main body are engaged.

2. A device for preventing slippage of protective covers in accordance with Claim 1, in which said main body is formed from a base (16) and an engaging piece (17) and an engaged piece (18), which extend from the sides of said base; said engaged piece having hook-shaped engaging pieces (18b) on a front end of said engaged piece; said pair of insertion holes (12, 13) are placed on the boundary part between said base and said engaging piece and the boundary part between said base and said engaged piece on said main body; said pressing part is placed on at least one of said engaging piece and said engaged piece, or said base; and when said engaging pieces have been engaged, said pressing part presses the endoscope which is covered by said protective cover onto said base or at least one of said engaging piece and said engaged piece.

3. A device for preventing slippage of protective covers in accordance with Claim 1 or Claim 2, in which a projecting part (14, 34) is formed in a part of the inner surface of said main body which opposes said pressing part, such that when the endoscope which is covered by said protective cover is inserted and the main body ends joined, the protective cover is pressed into contact with said projecting part by the pressure of said pressing part (15,35).

4. A device for preventing slippage of protective covers in accordance with any of Claims 1 to 3, in which said pressing part (15) is formed by a flexible piece the tip of which is formed as a free end.

5. A device for preventing slippage of protective covers in accordance with any of Claims 1 to 3, in which said pressing part (35) is formed by a flexible piece both ends of which are fixed to the inner surface of said main body and the center part of which is curved and projects outward.

6. A device for preventing slippage of protective covers in accordance with any of Claims 1 to 5, in which said pressing part (15, 35) is formed from a resin.

7. An endoscope set which comprises an endoscope (20), a protective cover (25), and a device (10, 30) for preventing slippage of protective covers in accordance with any of Claims 1 to 6.

## Patentansprüche

1. Vorrichtung (10, 30) zur Verhinderung des Abrutschens von Schutzabdeckungen für Endoskope relativ zum Endoskop, wobei die Vorrichtung Folgendes aufweist:
einen flexiblen plattenförmigen Hauptkörper (11), dessen beide Enden durch Biegen desselben verbunden werden können; und ein Paar Einführungslöcher (12, 13), die in diesem Hauptkörper mit einem Abstand dazwischen angebracht sind und durch die ein Endoskop, das mit der Schutzabdeckung abgedeckt ist, eingeführt werden kann; **dadurch gekennzeichnet, dass** die Vorrichtung ferner Folgendes umfasst: einen flexiblen Druckabschnitt (15, 35), der auf der Innenseite des Hauptkörpers sitzt und das Endoskop, das mit der Schutzabdeckung abgedeckt ist, verformen und auf einen anderen Abschnitt (14) in der Innenseite des Hauptkörpers drücken kann, wenn die Enden des Hauptkörpers in Eingriff miteinander stehen.

2. Vorrichtung zur Verhinderung des Abrutschens von Schutzabdeckungen nach Anspruch 1, bei der der Hauptkörper aus einem Boden (16) und einem eingreifenden Stück (17) sowie einem ergriffenen Stück (18), die sich von den Seiten des Bodens erstrecken, geformt ist, wobei das ergriffene Stück hakenförmige eingreifende Stücke (18b) auf einem Vorderende des ergriffenen Stücks aufweist, wobei das Paar von Einführungslöchern (12, 13) auf dem Grenzteil zwischen dem Boden und dem eingreifenden Stück und dem Grenzteil zwischen dem Boden und dem ergriffenen Stück auf dem Hauptkörper angeordnet ist, wobei der Druckabschnitt auf dem eingreifenden Stück und/oder dem ergriffenen Stück oder dem Boden angeordnet ist, und wobei der Druckabschnitt das Endoskop, das mit der Schutzabdeckung abgedeckt ist, auf den Boden oder das eingreifende Stück und/oder das ergriffene Stück drückt, wenn die eingreifenden Stücke in Eingriff stehen.

3. Vorrichtung zur Verhinderung des Abrutschens von Schutzabdeckungen nach Anspruch 1 oder Anspruch 2, bei der ein vorstehender Teil (14, 34) in einem Teil der Innenseite des Hauptkörpers, der dem Druckabschnitt gegenüberliegt, geformt ist, so dass die Schutzabdeckung durch den Druck des Druckabschnitts (15, 35) in Berührung mit dem vorstehenden Teil gedrückt wird, wenn das Endoskop, das mit der Schutzabdeckung abgedeckt ist, eingeführt wird und die Enden des Hauptkörpers verbunden sind.

4. Vorrichtung zur Verhinderung des Abrutschens von Schutzabdeckungen nach einem der Ansprüche 1 bis 3, bei der der Druckabschnitt (15) aus einem flexiblen Stück geformt wird, dessen Spitze als freies Ende geformt ist.

5. Vorrichtung zur Verhinderung des Abrutschens von Schutzabdeckungen nach einem der Ansprüche 1 bis 3, bei der der Druckabschnitt (35) aus einem flexiblen Stück geformt wird, dessen beide Enden an der Innenseite des Hauptkörpers befestigt sind und dessen Mittelteil gebogen ist und nach außen vorsteht.

6. Vorrichtung zur Verhinderung des Abrutschens von Schutzabdeckungen nach einem der Ansprüche 1 bis 5, bei der der Druckabschnitt (15, 35) aus einem Harz geformt wird.

7. Endoskopset aus einem Endoskop (20), einer Schutzabdeckung (25) und einer Vorrichtung (10, 30) zur Verhinderung des Abrutschens von Schutzabdeckungen nach einem der Ansprüche 1 bis 6.

## Revendications

1. Dispositif (10, 30) pour empêcher que des couvercles protecteurs d'endoscope ne glissent par rapport audit endoscope, le dispositif étant pourvu des éléments suivants :
un corps principal (11) flexible en forme de plaque dont les deux extrémités peuvent être jointes par flexion ;
une paire de trous d'insertion (12, 13) réalisés dans ledit corps principal à une certaine distance l'un de l'autre et à travers lesquels un endoscope recouvert par ledit couvercle protecteur peut être inséré ;
**caractérisé en ce que** le dispositif comprend en outre :
une pièce de pression flexible (15, 35) qui est placée sur la surface intérieure dudit corps principal et qui est agencée pour déformer et pour comprimer l'endoscope recouvert par ledit couvercle protecteur sur une autre partie (14) prévue dans la surface intérieure dudit corps principal lorsque les extrémités dudit corps principal sont emboîtées.

2. Dispositif pour empêcher le glissement de couvercles protecteurs selon la revendication 1, dans lequel ledit corps principal est formé à partir d'une base (16) et d'une pièce d'emboîtement (17) et d'une pièce emboîtée (18) qui s'étendent depuis les côtés de ladite base ; ladite pièce emboîtée ayant des pièces d'emboîtement (18b) en forme de crochet sur une extrémité frontale de ladite pièce emboîtée ; ladite paire de trous d'insertion (12, 13) est placée sur la partie de délimitation prévue entre ladite base et ladite pièce d'emboîtement et la partie de délimitation entre ladite base et ladite pièce emboîtée est placée sur ledit corps principal ; ladite pièce de pression est placée sur au moins un élément parmi ladite pièce d'emboîtement et ladite pièce emboîtée, ou ladite base ; et lorsque lesdites pièces d'emboîtement ont été emboîtées, ladite pièce de pression comprime l'endoscope qui est recouvert par ledit couvercle protecteur sur ladite base ou sur au moins un élément parmi ladite pièce d'emboîtement et ladite pièce emboîtée.

3. Dispositif pour empêcher le glissement de couvercles protecteurs selon la revendication 1 ou 2, dans lequel une partie saillante (14, 34) est formée dans une partie de la surface intérieure dudit corps principal qui est opposée à ladite pièce de pression, de telle sorte que lorsque l'endoscope recouvert par ledit couvercle protecteur est inséré et que les extrémités du corps principal se rejoignent, le couvercle protecteur est pressé pour venir en contact avec ladite partie saillante du fait de la pression de ladite pièce de pression (15, 35).

4. Dispositif pour empêcher le glissement de couvercles protecteurs selon l'une quelconque des revendications 1 à 3, dans lequel ladite pièce de pression (15) est formée par une pièce flexible dont l'embout prend la forme d'une extrémité libre.

5. Dispositif pour empêcher le glissement de couvercles protecteurs selon l'une quelconque des revendications 1 à 3, dans lequel ladite pièce de pression (35) est formée par une pièce flexible dont les deux extrémités sont fixées à la surface intérieure dudit corps principal et dont la partie centrale est incurvée et ressort vers l'extérieur.

6. Dispositif pour empêcher le glissement de couvercles protecteurs selon l'une quelconque des revendications 1 à 5, dans lequel ladite pièce de pression (15, 35) est moulée à partir d'une résine.

7. Ensemble endoscope comprenant un endoscope (20), un couvercle protecteur (25) et un dispositif (10, 30) pour empêcher le glissement de couvercles protecteurs selon l'une quelconque des revendications 1 à 6.
